# EUROPEAN PATENT SPECIFICATION

(11) **EP 4 124 305 B1**
(45) Date of publication and mention of the grant of the patent: **20.11.2024**
(21) Application number: 22187383.9
(22) Date of filing: 28.07.2022
(51) Int. Cl.: A61B 17/221, A61M 25/00, A61B 17/22, A61M 25/06

(54) **COLLAPSIBLE SUPER-BORE CATHETER**
ZUSAMMENKLAPPBARER KATHETER MIT SUPERBOHRUNG
CATHÉTER SUPER-ALÉSAGE PLIABLE

(30) Priority: 29.07.2021 US 202163203714 P
(43) Date of publication of application: 01.02.2023
(73) Proprietor: Neuravi Limited, Galway H91 K5YD (IE)
(72) Inventor: VALE, David, Galway, H91 K5YD (IE); KELLY, Ronald, Galway, H91 K5YD (IE); KEATING, Karl, Galway, H91 K5YD (IE); CASEY, Brendan, Galway, H91 K5YD (IE)
(74) Representative: Carpmaels & Ransford LLP

(56) References cited:
- EP-A1- 3 705 066
- WO-A1-2021/016213
- US-A1- 2020 205 845
- US-A1- 2021 153 884
- US-A1- 2021 154 433

## Description

### Field of Invention

The present invention generally relates devices for removing acute blockages from blood vessels during intravascular medical treatments. More specifically, the present invention relates to retrieval catheters with expandable tips into which an object or objects can be retrieved.

### Background

Clot retrieval aspiration catheters and devices are used in mechanical thrombectomy for endovascular intervention, often in cases where patients are suffering from conditions such as acute ischemic stroke (AIS), myocardial infarction (MI), and pulmonary embolism (PE). Accessing the neurovascular bed in particular is challenging with conventional technology, as the target vessels are small in diameter, remote relative to the site of insertion, and highly tortuous. Traditional devices are often either too large in profile, lack the deliverability and flexibility needed to navigate particularly tortuous vessels, or are not effective at removing a clot when delivered to the target site.

Many existing designs for aspiration retrieval catheters are often restricted to, for example, inner diameters of 6Fr or between approximately 1.73 - 1.88mm (0.068 - 0.074 inches). Larger sizes require an even larger guide or sheath to be used, which then necessitates a larger femoral access hole to close. Most physicians would prefer to use a 2.67mm (8F) guide / 2mm (6F) sheath combination, and few would be comfortable going beyond a 3mm (9F) guide / 2.33mm (7F) sheath combination. This means that once at the target site, a clot can often be larger in size than the inner diameter of the aspiration catheter and must otherwise be immediately compressed to enter the catheter mouth. This compression can lead to bunching up and subsequent shearing of the clot during retrieval. Firm, fibrin-rich clots can also become lodged in the fixed-mouth tip of these catheters making them more difficult to extract. This lodging can result in softer portions breaking away from firmer regions of the clot.

Small diameters and fixed tip sizes are also less efficient at directing the aspiration necessary to remove blood and thrombus material during the procedure. The suction must be strong enough such that any fragmentation that may occur as a result of aspiration or the use of a mechanical thrombectomy device cannot migrate and occlude distal vessels. When aspirating with a fixed-mouth catheter, a significant portion of the aspiration flow ends up coming from vessel fluid proximal to the tip of the catheter, where there is no clot. This significantly reduces aspiration efficiency, lowering the success rate of clot removal.

Large bore intermediate and aspiration catheters and/or those with expandable tips are therefore desirable because they provide a large lumen and distal mouth to accept a clot with minimal resistance. The bore lumen of these catheters can be nearly as large as the guide and/or sheath through which they are delivered, and the expandable tip can expand to be larger still. When a clot is captured and drawn proximally into a tip with a funnel shape, the clot can be progressively compressed during retrieval so that it can be aspirated fully through the catheter and into a syringe or cannister. The expandable tips can be, for example, an underlying metallic support frame made from a suitably elastic material so that it can expand when deployed from the guide and/or sheath. The frame can be shaped to provide a clinically atraumatic vessel crossing profile with the ability to maintain an inner diameter and recover its shape when displaced laterally. A polymeric jacket can cover the frame to provide a soft outer layer for catheter advancement. Alternatively, the expandable tip can be of fully polymeric construction of one or multiple materials. However, designs with fixed braids, frames, or expanded sizes do not allow for any additional expansion of the tip which can complicate the interaction with and reception of a clot during ingestion.

Combining the clinical needs of these catheters without significant tradeoffs can be tricky, and many contemporary designs fall short in a number of categories. Catheter designs attempting to overcome the above-mentioned design challenges would need to have a large bore and an expandable tip with sufficient hoop strength in the expanded state to resist aspiration forces without collapse while having a structure capable of folding down consistently and repeatably when retrieved into an outer guide and/or sheath. The tip structure needs to have the flexibility and elasticity to survive the severe mechanical strains imparted when navigating the tortuous vasculature, while also being capable of expanding elastically as a clot is ingested for better interaction with and retention of the clot.

The size and shape of other expandable devices often means they are limited to being deployed directly at a target occlusion. Tip designs disclosed herein can maintain good pushability but are also sized to be advanced within an outer sheath, and then in the expanded state in a vessel with minimal tendency to further over-expand outward when placed in compression. Such over-expansion can increase the delivery forces for the catheter and can also result in the aspiration catheter binding within the outer guide or sheath through which it is delivered. Further expansion in a vessel can damage vessel walls or snag when advanced.

The present designs are aimed at providing an improved retrieval catheter with an expansile tip capable of addressing the above-stated deficiencies. WO2021/016213A1 describes devices and methods for aspiration of thrombus. EP3705066A1 describes an actuated clot retrieval catheter. US2021/154433A1 describes an clot retrieval device with outer sheath and inner catheter. US2020/205845A1 describes devices and methods for removing obstructive material from an intravascular site. US2021/153884A1 describes an actuated expandable mouth thrombectomy catheter.

### Summary

The invention is defined by claim 1. Embodiments of the invention are defined by the dependent claims. It is an object of the present designs to provide devices and methods to meet the above-stated needs. The designs can be for a clot retrieval catheter capable of remove a clot from cerebral arteries in patients suffering AIS, from coronary native or graft vessels in patients suffering from MI, and from pulmonary arteries in patients suffering from PE and from other peripheral arterial and venous vessels in which a clot is causing an occlusion.

In some examples, a device can be a large bore catheter having a proximal elongate body with a proximal end, a distal end, an internal lumen, and a longitudinal axis extending therethrough. The elongate body can terminate at a distal expansile tip, which can be integrally-formed or fixedly connected. The tip can have a support frame with a framework of struts configured to be expanded and collapsed between a collapsed delivery configuration and an expanded deployed configuration when the catheter is advanced and retracted from an outer guide and/or sheath at the site of an occlusive thrombus.

In some examples, the support frame can have a longitudinal length sized to be less than twice the inner diameter of the elongate body in the expanded deployed configuration. In other examples, the longitudinal length can be less than three times the inner diameter of the elongate body in the expanded deployed configuration.

The support frame can also have a range of inner and outer diameters. In one example, the support frame can have a maximum inner diameter (or maximum outer diameter) in the expanded state equal to or less than the diameter of a target vessel just proximal of a target clot. In another example, the expanded support frame can be sized to have a larger inner diameter than the inner diameter of an outer sheath through which it is delivered. In other examples, the support frame can have an inner diameter of approximately 1.78mm (0.070") in the collapsed delivery configuration and a maximum inner diameter in a range of approximately 2.03 - 3.05mm (0.080 - 0.120 inches) in the expanded deployed configuration.

In further designs, the difference between the inner diameters of the support frame in the expanded deployed configuration and the collapsed delivery configuration can be more than 10% of the inner diameter in the collapsed delivery configuration. In similar designs, the difference between the inner diameters of the support frame in the expanded deployed configuration and the collapsed delivery configuration can be more than 20% of the inner diameter in the collapsed delivery configuration.

The framework of struts has one or more hoop segments and a plurality of deformable cells. The hoop segments and cells can be configured in such a way to balance the overall flexibility and radial force the frame is capable of. The hoop segments and cells can be joined directly or connected through one or more axial spines which can linking the support frame with the elongate body.

The cells can be arranged in a variety of ways within the support frame. In one example, there can be a single axial row of cells on one side of the frame, the remainder of the frame circumference made up of the hoop segments. In other example there can be two rows of axial cells configured 180 degrees apart, or at some other orientation, with hoop segments linking the rows.

The cells are configured to assume an axially extended profile elongated parallel to the longitudinal axis when the support frame is in the collapsed delivery configuration; and expand to a radially extended profile elongated normal to the longitudinal axis when the support frame is in the expanded deployed configuration. In this way, the cells take up a very small percentage of the circumference of the support frame when collapsed, and a substantially larger percentage when expanded. This means that the one or more hoop segments account for a greater percentage of the inner diameter when the support frame is collapsed than when it is expanded. In some instances, the cells can make up less than 30% of the circumference of the support frame when the frame is in the collapsed delivery configuration. More preferably, the cells would constitute less than 20% of the circumference in the collapsed configuration.

The orientation of the struts of the deformable cells can be used to express the degree to which they are collapsed or expanded. In some examples, the struts of the deformable cells can form an angle with the longitudinal axis that is less than 30 degrees in the collapsed delivery configuration. Similarly, in other examples when the frame is in the expanded deployed configuration, the struts of the deformable cells can form an angle with the longitudinal axis that is greater than 45 degrees.

The cells can also have an arc length forming a proportion of the circumference which is significantly smaller when the cells and tip are in the collapsed state than when in the deployed expanded state. For example, the struts of the deformable cells can have a first arc length in the collapsed delivery configuration less than a second arc length in the expanded deployed configuration. Similarly, since the cells of the support frame occupy a small percentage of the support frame circumference when in the collapsed delivery configuration, this can mean that the one or more hoop segments account for a greater percentage of the inner diameter when the support frame is collapsed than when it is expanded.

Another measure of the taper of the funnel shape of the tip formed by expansion of the deformable cells can be determined by the flare angle formed by the contour of the tip with the longitudinal axis. In some examples, at least a portion of the support frame can form a flare angle with the longitudinal axis, while other portions may remain substantially parallel with the axis when expanded. The flare angle can be configured to be approximately horizontal (0 degrees) in the collapsed delivery configuration and approximately vertical (90 degrees) in the expanded deployed configuration when the deformable cells are expanded.

In some examples, a method for manufacturing a catheter can be disclosed. The method is defined by claim 13

In some examples, the chilled temperature can be more ideally below the Martensitic finish temperature. The method can then include removing the second oversized mandrel from the support tube.

The outer jackets can be made from or coated with an elastomer or similar material to provide a low-friction surface to facilitate navigation within blood vessels as well as other catheters.

In some examples, the soft elastic jacket of the distal tip section can be fused with the one or more proximal outer polymer jackets of the elongate body. A further step can involve forming a flexible polymeric lip extending radially from the distal elastic jacket of the distal tip. With the stepped mandrel removed, an additional step can include applying an inner hydrophilic coating to the interior and exterior of the distal tip assembly.

Other aspects of the present disclosure will become apparent upon reviewing the following detailed description in conjunction with the accompanying figures. Additional features or manufacturing and use steps can be included as would be appreciated and understood by a person of ordinary skill in the art.

### Brief Description of the Drawings

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation. It is expected that those of skill in the art can conceive of and combine elements from multiple figures to better suit the needs of the user.
FIG. 1 is a view of a large bore clot retrieval catheter with an expandable tip configured for being advanced through the vasculature according to aspects of the present invention;
FIG. 2A illustrates a large bore clot retrieval catheter with an expandable tip in the collapsed delivery configuration according to aspects of the present invention;
FIG. 2B shows the large bore clot retrieval catheter of FIG. 2A with the expandable tip in the expanded deployed configuration according to aspects of the present invention;
FIG. 3A depicts a flat pattern of an example support frame in the expanded deployed configuration according to aspects of the present invention;
FIG. 3B shows a portion of the flat pattern of FIG. 3A in the collapsed delivery configuration according to aspects of the present invention;
FIG. 4 illustrates an assembled tip similar to FIG. 3 according to aspects of the present invention;
FIGs. 5A-5B are another example of an elongate body and a support frame of a large bore clot retrieval catheter in the collapsed and expanded configurations, respectively, according to aspects of the present invention;
FIGs. 6A-6B are views of another example of an elongate body and a support frame in the collapsed and expanded configurations, respectively, according to aspects of the present invention;
FIGs. 7A-7F illustrate steps of a method for the construction of a catheter according to aspects of the present invention;
FIG. 8 is a flow diagram of the steps from FIGs. 7A-7F according to aspects of the present invention; and
FIG. 9 shows a flow diagram for a method of use for a catheter (method not claimed) in treating a patient with a vessel occlusion according to aspects of the present invention.

### Detailed Description

Specific examples of the present invention are now described in detail with reference to the Figures, where identical reference numbers indicate elements which are functionally similar or identical. The examples address many of the deficiencies associated with traditional clot retrieval aspiration catheters, such as poor or inaccurate deployment to a target site and ineffective clot removal.

The designs herein can be for a clot retrieval catheter with a large bore lumen and a distal tip that can expand to a diameter larger than that of the guide or sheath through which it is delivered when advanced beyond the distal end. The designs can have a proximal elongate body for the shaft of the catheter, and a distal tip with an expanding metallic support frame and outer polymeric jacket to give the tip atraumatic properties and the ability to flexibly expand further in the expanded deployed configuration when ingesting a clot. The support frame can be designed so that the expanding/collapsing movement is focused a small portion of the circumference though a plurality of deformable cells that can collapse to be almost flat and parallel to the longitudinal axis for ease of deliverability, but expand with a very steep, nearly vertical angle for good resistance to collapse under aspiration. The catheter frame and tip can be sufficiently flexible to navigate highly tortuous areas of the anatomy and be able to recover and maintain the inner diameter of the lumen when displaced in a vessel.

Accessing the various vessels within the vascular, whether they are coronary, pulmonary, or cerebral, involves well-known procedural steps and the use of a number of conventional, commercially-available accessory products. These products, such as angiographic materials, mechanical thrombectomy devices, microcatheters, and guidewires are widely used in laboratory and medical procedures. When these products are employed in conjunction with the devices and methods (not claimed) of this invention in the description below, their function and exact constitution are not described in detail. While the description is in many cases in the context of thrombectomy treatments in intercranial arteries, the disclosure may be adapted for other procedures and in other body passageways as well.

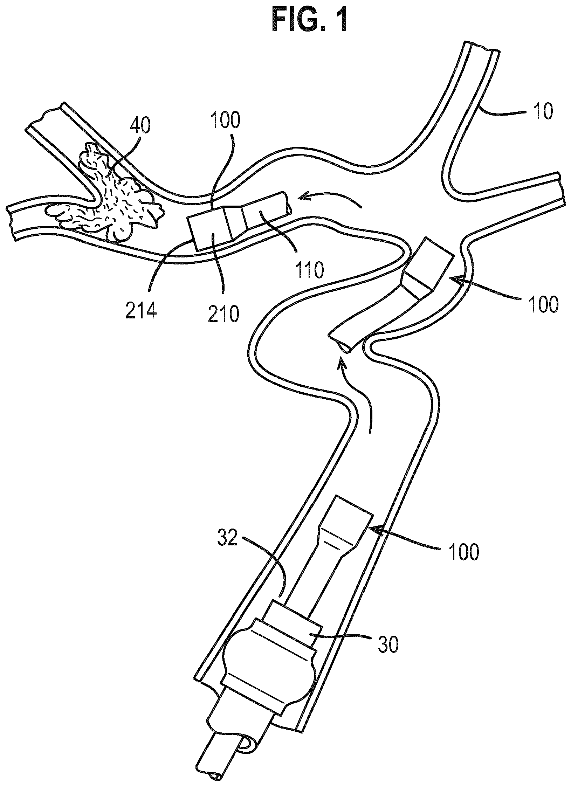

Turning to the figures, FIG. 1 illustrates a possible sequence for approaching an occlusive clot 40 using a large bore clot retrieval catheter 100 of the designs disclosed herein. The clot 40 can be approached with the catheter 100 collapsed within a guide sheath 30 or other access catheter. When the vasculature 10 becomes too narrow and/or tortuous for further distal navigation with the guide sheath 30, the catheter 100 can be deployed for further independent travel distally. The catheter 100 can be highly flexible such that it is capable of navigating the M1 or other tortuous regions of the neurovascular to reach an occlusive clot, and have an expanded outer diameter slightly less than that of the target vessel so the catheter is capable of distal navigation independently after deployment.

The clot retrieval catheter 100 can have a flexible elongate body 110 serving as a shaft with a large internal bore (which in some cases can be 2.29mm (0.080 inches) or larger) and a distal tip section having a collapsible support frame 210. The large bore helps the catheter to be delivered to a target site by a variety of methods. These can include over a guidewire, over a microcatheter, with a dilator/access tool, or by itself.

In many cases, the design of the tip can be configured so that the entire catheter 100 can be delivered through (and retrieved back through) common standard 6F sheaths/8F guides, which typically have inner lumens of less than 2.29mm (0.090 inches). The tip can self-expand once advanced to an unconstrained position distal to the distal end 32 of the guide sheath 30. As the catheter can be deployed proximal of and then be advanced independently to a remote occlusion, the support frame 210 of the tip is designed to be able to resist collapse from the forces of aspiration, have excellent lateral flexibility in both the expanded and collapsed states, and an atraumatic profile to prevent snagging on bifurcations in vessels.

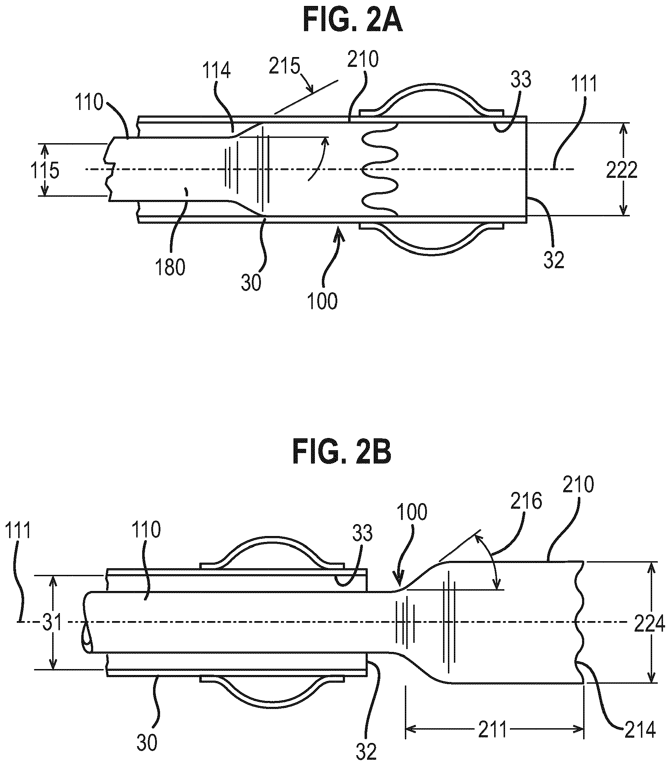

FIG. 2A and FIG. 2B show a closer view of the distal region of the catheter 100 of FIG. 1 confined within, and then delivered beyond, the guide sheath 30. The catheter 100 can have a tubular elongate body 110 configured around a longitudinal axis 111. The structure for the elongate body 110 can define the catheter lumen, which can be used for the delivery of auxiliary devices, contrast injection, and direct distal aspiration to a clot face. The underlying structure of the elongate body 110 can be, for example, a frame cut from a hypotube of a superelastic material with shape memory properties, such as Nitinol with an internal low friction liner and outer polymer jacket or jackets 180 that can be reflowed into the structure during manufacturing. Alternately, a more traditional polymer and/or metal braid or coil support structure can be used, or some combination of these.

The outer surface of the elongate body 110 and support frame 210 can be at least partially covered by an outer jacket or jackets 180. The jacket 180 can block proximal fluid from entering the expanded tip during aspiration and retrieval of the clot, allowing for more efficient direction of the aspiration force while preventing the distal migration of clot fragments or other debris during the procedure. In one example, the jacket 180 can be formed from a highly-elastic material such that the radial force exerted by expanding the expansile tip is sufficient to stretch the membrane to the funnel shape contours of the tip when in the expanded deployed configuration. One example can be using a ductile elastomer which has the advantages of being soft and flexible with resistance to tearing and perforation due to a high failure strain. Alternately, the jacket can be baggy and loose and fold over the support frame edges so that the frame can move more freely when expanded and collapsed.

As mentioned, the elongate body can be sized to be compatible with relatively low-profile access sheaths and catheters, so that a puncture wound in the patient's groin (in the case of femoral access) can be easily and reliably closed. For example, the clot retrieval catheter 100 can be required to pass through the lumen 33 of a sheath or guide 30 with an inner diameter 31 of less than 2.80 mm (0.110 inches), preferably 2.29mm (0.090 inches), in some cases less than 2.21mm (0.087 inches), and most preferably less than 2.16mm (0.085 inches). In other examples with a 5Fr sheath, the inner diameter can be less than 2.03mm (0.080 inches) and can be as small as 1.78mm (0.070 inches). Therefore, the catheter shaft can have an overall delivery profile with an inner diameter 115 of approximately 1.70 - 1.88mm (0.067 - 0.074 inches) (0.084 inch or 2 mm outer diameter), and yet be able to expand its distal tip and mouth to a size just smaller than the diameter of the vessel approximate where the clot is located, which could be as large as 5 mm. It can be appreciated that larger or smaller catheter shafts can be used if paired with a larger or smaller guide/sheath.

The support frame 210 can similarly be sized for compatibility. The support frame 210 can have an inner diameter 222 in the collapsed delivery configuration sized so that radial forces are not excessively high for delivery through the selected guide sheath 30, which can for example have an inner diameter of 2.16mm (0.085 inches). Once deployed beyond the distal end 32 of the guide sheath 30, the support frame 210 can grow radially such that the tip is just smaller in diameter than the target vessel. For example, a large size of approximately 3-5 mm for clots in the ICA, an intermediate size for proximal / large M! clots of approximately 2.29 - 2.80mm (0.090 - 0.110 inches) in diameter, and/or a small size for small/distal M1 clots of approximately 2.03 - 2.29mm (0.080 - 0.090 inches) in diameter. In many situations, the tip can have a maximum inner diameter 224 within a range of approximately 2.03 - 3.05mm (0.080 - 0.120 inches) in the expanded deployed configuration.

A relative measure of how steeply the funnel profiled portion of the support frame 210 of the expandable tip grows can be taken from an angle corresponding to the increase in size relative to increasing distal distance along the longitudinal axis 111. This flare angle can be separated into a collapsed angle 215 and an expanded angle 216 as seen in FIG. 2A and FIG. 2B, respectively. To meet the other design requirements of the expandable tip, such as compressive hoop strength and a resistance to over-expanding when placed in tension or compression during a procedure, the collapsed flare angle 215 would ideally be as close to 0 degrees as possible for deliverability and column strength. Similarly, when the frame is in the expanded deployed configuration the expanded flare angle 216 would ideally be as close to 90 degrees (or vertical with respect to the axis 111) as possible for good radial force capacity.

To meet these requirements, the overall length of the frame 210, as measured from the distal end 114 of the elongate body 110 to the distal end 214 of the frame, can also be kept as short as practical. In some examples, this longitudinal length 211 can be less than twice, or preferably less than three times, the inner diameter 115 of the elongate body. A short frame 210 can maintain good hoop stiffness while maintaining excellent flexibility through having a short lever distance to hinge off the elongate body 110. A short frame can also be tailored to minimize stretch and deformation in the outer polymer jackets.

In some examples, specifications for the frame 210 can include that the expanded tip has a "crush" strength (utilizing a flat plate squash test) of greater than a specified value (or within a range of values) in the expanded configuration. Alternately, or in addition, the design can be specified to have a radial force of less than a specified value (or within a range of values) in the collapsed/delivery configuration.

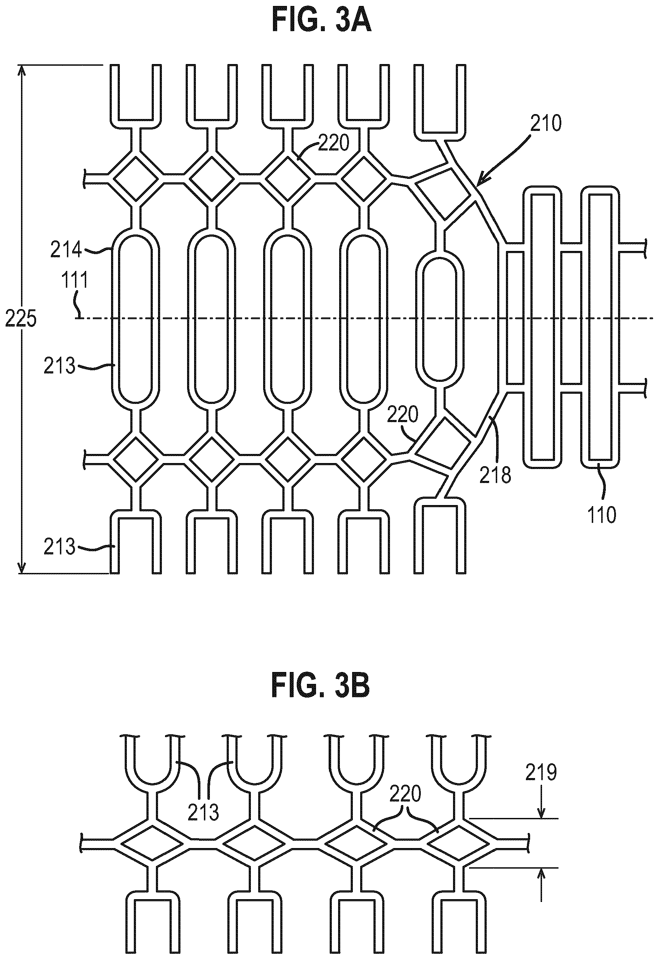

A developed view of an example laser cut pattern for an example support frame 210, post-expansion, for the large bore catheter is depicted in FIG. 3A. The more proximal portion of the support frame 210 can be cut to the diameter of the elongate body 110 (or cut integrally from the same hypotube), while the more distal portion can be expanded to a circumference dimension 225 at the distal end 214 which would yield the desired expanded inner diameter of the support frame. The frame 210 can be a laser cut Nitinol or steel network of interconnected struts. The network of struts can be relatively dense to provide good scaffolding for the outer jackets, especially in regions where a particularly soft and highly flexible polymer or blend is chosen.

The network of struts making up the support frame 210 has two axial series of deformable cells 220 connected by hoop segments 213. The deformable cells 220 are capable of elongating circumferentially with respect to the axis 111 when the frame is in radially expanded to the deployed configuration, and elongating axially (flattening) with respect to the longitudinal axis when the support frame is reduced to the collapsed delivery configuration for advancement (or when the frame is withdrawn back into an outer sheath). The cells 220 can be substantially diamond shape to allow the vertices to facilitate more uniform deformation/expansion as shown or take some other profile such as a rounded or ovular shape.

When the support frame 210 is in the expanded deployed configuration to elongate the cells 220 circumferentially, the cell struts assume a steep, more vertical orientation with respect to the longitudinal axis 111. This gives the frame better compressive hoop strength in an expanded state to resist collapse of the tip under aspiration. Additionally, steeper cell angles can reduce the tendency of the support frame to further expand radially or over-expand when the catheter 100 is in compression being advanced through a vessel independently (after deployment from the guide sheath) with the frame in the expanded state. This can help the expanded tip to avoid snagging in vessels, particularly in tight bends and when being pushed through vessels of progressively smaller diameters.

FIG. 3B shows an inset view of a sample configuration for the deformable cells 220 when the support frame 210 is in the collapsed delivery configuration. When flattened, the struts of the cells 220 can assume a shallow, almost horizontal angle to give the support frame 210 more column stiffness and pushability. Further, shallow angles can resist the tendency of the frame to expand when the catheter is advanced through the guide and/or sheath in the collapsed state. This resistance can help to prevent binding and high delivery forces during advancement.

Since the hoop segments 213 are of fixed size and do not expand, the change in tip circumference (and thus diameter) due to the expansion of the support frame 210 is due in its entirety to the elongation of the deformable cells 220. As a result, it is desirable for the cells to fold as flat as possible (and account for as little of the total circumference of the frame) in the collapsed delivery configuration for peak performance. Similarly, it is preferred that the cells come together to be near vertical members when in the expanded deployed configuration. Therefore, since the hoop segments 213 are of fixed size, the percentage of the inner diameter 222 of the support frame 210 accounted for by the segments in the collapsed delivery configuration is greater than the percentage of the expanded inner diameter 224 accounted for by the hoop segments in the deployed configuration.

The axial series of deformable cells 220 as shown in FIG. 3A and FIG. 3B are arranged in a parallel rows with respect to the longitudinal axis 111, but it can be appreciated that they can be offset circumferentially from one another, even by 90 degrees or more within a lattice. Similarly, a single circumferential strut could be used to connect the cells 220, and the hoop segments 213 could be axially offset into the interstitial spaces between these struts. Ideally, the pattern of the cells 220 and hoop segments 213 can be symmetric about the axis 111 so that expansion and collapse/wrap down of the support frame 210 can be consistent and repeatable. When the tip is in the collapsed state, the struts of the cells 220 can form an arc length 219 that is a smaller overall component of the tip circumference.

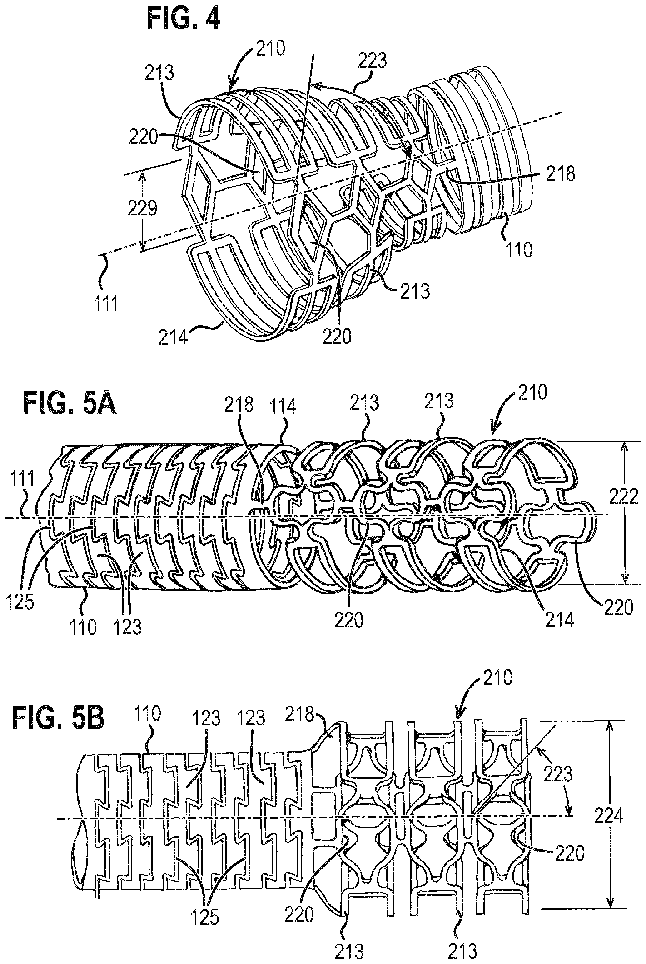

The funnel shape of an expanded support frame 210 similar to that of FIG. 3A is illustrated in FIG. 4. This profile can have a gradual taper as shown so that a captured clot is progressively compressed proximally until there is little to no resistance to clot entry into the elongate body 110. The assembled support frame 210 can have a smooth, low friction inner surface without any sagging of the outer jacket (not shown) or other structural protrusions into the interior during aspiration.

The elongate body 110 can have a number of forms and, as mentioned, can be formed integrally with the support frame 210 by laser cutting the same hypotube. One or more connecting spines 218 can be used to link the body and the tip. The spine or spines 218 can add stiffness and define planes or axis about which the support frame can bend relative to the expansile body. Spines can have a fixed, rigid connections as shown or can be of a form which allows more flexing such as a waveform shape or threaded eyelet.

The shape of the deformable cells 220 can also vary, so long as apices, creases, or other biasing features exist for reliable expansion and folding. The cells can have an arc length 219, 229 forming a proportion of the circumference in the collapsed and deployed states. As mentioned, the struts of the cells can form a smaller first arc length 219 in the collapsed delivery configuration and a larger second arc length 229 when the tip is expanded. In many cases, the cell arc length can expand by at least 50%, but preferably up to 100% or more between the collapsed and expanded configurations. In some examples, the arc length can increase by as much as 200% or more between the collapsed and expanded states. When in the deployed configuration, some additional circumferential deformation capacity of the cells, which would increase the cell expanded arc length 229, can allow the support frame to have additional flexibility to expand to the contours of a large or firm, fibrin-rich clot as it is ingested. This added expansion allows for better clot management and reduces the risk of shearing when compared to other tips with stiffer, less compliant frames or those utilizing harder polymeric materials.

The struts of the cells 220 can form different angles with the longitudinal axis 111 in the collapsed delivery configuration and the expanded deployed configuration. As the cells are the primary deforming members between the states of the frame 210, the angle achieved in one configuration (either collapsed or expanded) influences what can be achieved in the other, so a design balance for performance must be struck.

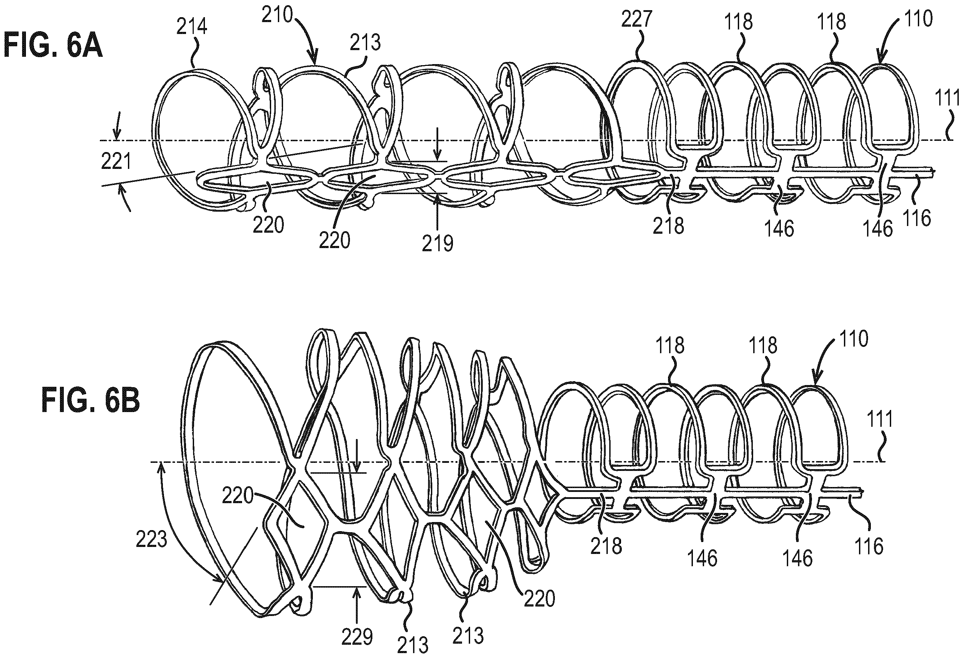

As mentioned, it is desirable to have the deformable cells 220 deform or fold as flat (or horizontal) as possible when the frame is in the collapsed delivery configuration. If the collapsed angle (see, for example, angle 221 in FIG. 6) is too high, the radial force component can be significant, and the catheter can bind or otherwise become undeliverable. Ideally this collapsed angle would be nearly zero but is more realistically less than 30 degrees and ideally less than 20 degrees with respect to the longitudinal axis 111. This shallow angle allows the cells 220 to play a role in force transmission (with axial and radial components) from the user to the expandable tip.

The cell angle 223 when the support frame 210 is in the expanded deployed configuration as seen in FIG. 4 would preferably be as close to 90 degrees as possible with the axis 111 to maximize the radial force capabilities of the frame. A steep angle can allow for some additional frame expansion without overexpansion when being advanced through a vessel. The expanded cell angle 223 can be greater than 45 degrees, but ideally greater than 60 degrees and most preferably greater than 70 degrees. Expansion and folding of the frame can function at lower angles, but increasingly steep angles can give the frame greater hoop strength.

Another example of an expandable support frame 210 and elongate body 110 are shown in FIG. 5A and FIG. 5B. The support frame and the elongate body can be cut from the same hypotube of a shape memory, superelastic alloy such that they are concentric with the longitudinal axis 111 and share the same collapsed inner diameter 222 for smooth delivery through an outer sheath. Alternatively, the two structures can be laser cut separately and the one or more connecting spines 218 welded or otherwise adhered at the distal end 114 of the elongate body 110.

The number of connecting spines 218 used can be varied. Additional spines can increase axial stiffness and pushability, at the cost of decreased lateral stiffness. The use of additional spines 218 can also give the catheter greater resistance to localized elongation between the elongate body 110 and the support frame 210 when the frame is subjected to lateral and tensile loads. Multiple spines also help the support frame resist longitudinal compression and shortening during deployment to ensure exact placement at a treatment site. The disposition of the spines can also encourage bending of the frame 110 in a single plane in certain planes as desired. For example, four connecting spines 218 clocked 90 degrees apart can aid in delivering a balanced and consistent push or thrust force through the length of the elongate body to the tip. This opposing spine arrangement can also prevent the frame 210 from bending in a direction circumferentially normal to a spine, a direction more prone to kinks or potentially fracture.

The underlying structure beneath the outer jackets for the elongate body 110 can have a ribbed, coil, braided, or other supporting frame or combination thereof. A configuration where radial slots create a puzzle-cut pattern of ribs from a hypotube is illustrated in FIG. 5A and FIG. 5B. The hypotube can be, for example, a 1.78mm (0.070 inch) inner diameter Nitinol tube with a wall thickness of 0.076mm (0.003 inches for delivery within a standard 2mm (6F) sheath / 2.67mm (8F) guide combination. The puzzle cut tube can be substantially a series of interlocking rib or ring segments 123. As each segment 123 is not fully integral with adjacent segments (either proximally or distally), the puzzle cut tube can twist about the longitudinal axis 111. A puzzle cut elongate body 110 construction can also resist tensile elongation and compressive shortening due to engagement of adjacent interlocking features 125. Distal facing interlocking features can engage a particular ring with the next distal ring, while proximal facing interlocking features can engage with the next proximal ring.

Flexibility of the puzzle cut elongate body 110 can be varied through the alteration of the interlocking geometry. The elongate body can, for example, lengthen by the sum of the spacing between the puzzle cut segments 123 distributed longitudinally. Similarly, allowable twist can be adjusted by altering the circumferential spacing between the segments 123 and their corresponding interlocking features 125. The twist properties offered by a puzzle cut design for the support tube can aid in the catheter bending and torqueing in multiple planes as it is advanced through tortuous vascular paths.

Similar to other designs, some examples can have a longitudinal spine (not shown) added internal or external to the puzzle cut structure of the elongate body 110. Other examples can have spines in the form of continuous uncut seams along the length of the hypotube. Spines can help prevent the puzzle cut tube from lengthening under tensile loads. In one example, a single spine, or twin spines spaced 180 degrees apart can have minimal impact on the ability of the puzzle rings to twist. The twist can change the preferred bending plane of the tube to a degree controlled by the designed allowable twist so that the support tube is capable of self-adjusting as it is advanced through tortuous vessels.

The support frame 210 of FIG. 5A and FIG. 5B can have opposing axial series of hoop segments 213 linking deformable cells 220. The cells 220 can have an oblong circular or ovular shape, with the oblong axis changing depending on whether the frame is in the collapsed delivery configuration (FIG. 5A) or the expanded deployed configuration (FIG. 5B). The cells can form two rows and be linked to the hoop segments 213 through horseshoe shaped joints where the legs of the horseshoe are spread when the frame is in the collapsed state. This shape can help the heat set support frame 210 to become more rounded funnel as opposed to being stretched into an ovular opening. The cells 220 can be sized for sufficient circumferential deformation so the support frame 210 can reach the desired inner diameter. In many cases this diameter can be approximately 2.03mm (0.080) inches but can depend on the diameters of target vessels and in some cases can be as large as approximately 3.05mm (0.120 inches). Other sizes, both larger and smaller, can be contemplated based on target location.

In another example, a single row of deformable cells 220 can form a backbone for the hoop segments 213 of the expandable support frame 210 in line longitudinally with an axial spine 116 of the elongate body 110. An example can be seen with the support frame 210 in the collapsed delivery configuration in FIG. 6A and in the expanded deployed configuration in FIG. 6B. A connecting spine strut 218 can provide a single support point between the support frame 210 and the distalmost rib 227 of the elongate body 110. When in bending, or when the tip is placed under compressive loads during retrieval of a clot, a single spine strut yields fewer rigid connections which can give the tip added flexibility and the ability to deflect locally for a tighter grip on the captured clot.

The support frame 210 can have broad looped hoop segments 213 extending between the row of diamond shaped deformable cells 220. The cells can expand from a nearly flat collapsed cell angle 221 of as close to zero degrees as-cut in FIG. 6A to the larger expanded cell angle 223 in FIG. 6B. Ideally the expanded cell angle is as close to 90 degrees as possible to maximize the radial force acting against collapse during aspiration. The expanding cell angles draw adjacent hoop segments closer together, shortening the length of the support frame 210 for better radial force and scaffolding of an outer jacket (not shown) in the deployed configuration. The struts of the cells forming the cell angles can be at least approximately 45 degrees, and more preferably greater than approximately 70 degrees with the tip in the expanded deployed configuration. The hoop segments 213 can have distally unconnected peaks 228 and do not have to be fixedly coupled to each other. Instead, the hoop segments can be interlaced to slide and fold relative to each other as the support frame 210 expands or contracts. Additional hoops segments 213 can be added to sacrifice some tip flexibility for additional radial force and support of the outer jackets. Similarly, fewer hoop segments can be utilized in situations where a jacket of greater stiffness or thickness requires less support.

Multiaxial flexibility of the catheter shaft can be improved by minimizing the overall number of connections between the support frame 210 and the spine or spines 116 of the elongate body 110. FIG. 6A illustrates the example where the elongate body framework has pairs of supporting ribs 118 which merge into a single spine connector 146 for connections with the spine 116. Each set of support ribs can have one, two, three, or more ribs 118. By connecting support ribs 118 in sets that have a single connection to the one or more spines 116, additional flexibility is gained by having a longer length of spine which is free to bend for a given density of ribs. This arrangement can also reduce strain at the connection between the spine connectors 146 and the axial spine 116.

Alternately, a similar design can see a series of supporting ribs 118 merging into diametrically opposed spine connectors for connections with twin spines 116 spaced 180 degrees apart. Additional spines can also be envisioned which trade some lateral flexibility for better pushability than can be achieved with fewer spines. The additional axial stiffness can also help prevent the elongate body from stretching under tension, such as when an expandable tip is being drawn proximally into an outer sheath with a firm clot.

Fewer connections between the tip and the spine 116 can give the framework of the elongate body 110 a greater ability to both bend and twist for a given density of support ribs 118 when compared to an elongate body where each rib has a direct connection to the one or more spines. It can also be appreciated that the ribs 118 of the elongate body 110 can be cut at an incline distal to their connection with the spine 116 to allow for the ribs to be reset to a position radially outward so the tube can be expanded during assembly on a mandrel or when clots with fibrin rich components are withdrawn through the catheter lumen.

Although the elongate body 110 shown in FIG. 6A and FIG. 6B is depicted as a laser cut hypotube, it is possible to transition in more proximal regions to a more conventional (and potentially less expensive) braided or coil structure. The braided structure can incorporate one or more axial spines as reinforcement so there is minimal tendency for the elongate body 110 to elongate under tension or compress when being advanced though an outer guide or sheath.

It should be noted that any of the herein disclosed catheters designs can also be used with one or more stentrievers. The combined stentriever retraction and efficient aspiration through the enlarged tip section in the expanded deployed configuration can act together to increase the likelihood of first pass success in removing a clot. The catheter can also direct the aspiration vacuum to the clot face while the stentriever holds a composite clot (comprised of friable regions and fibrin rich regions) together preventing embolization and aid in dislodging the clot from the vessel wall. The funnel-like shape of the tip section can also reduce clot shearing upon entry to the catheter and arrest flow to protect distal vessels from new territory embolization.

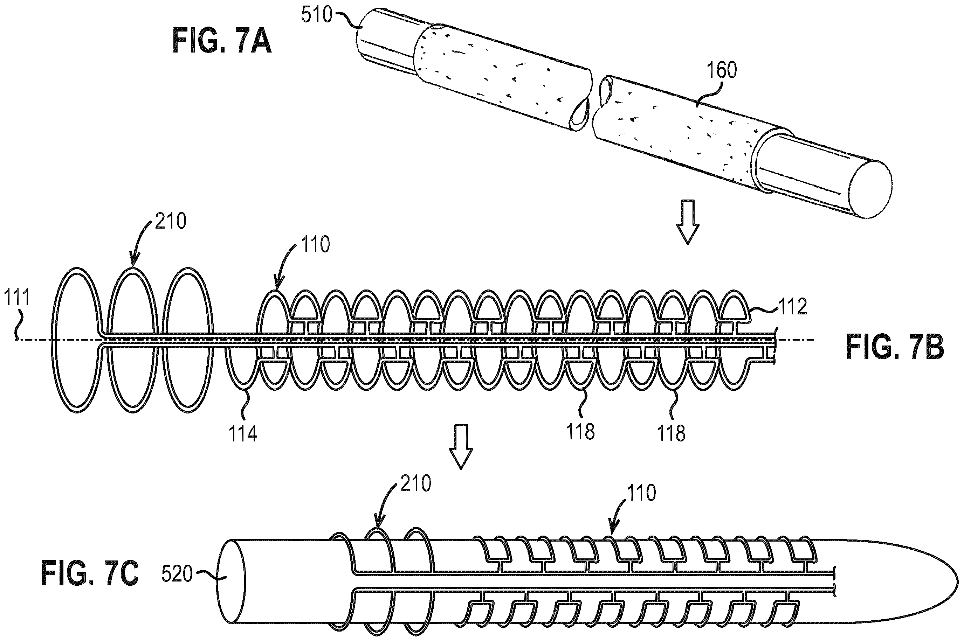

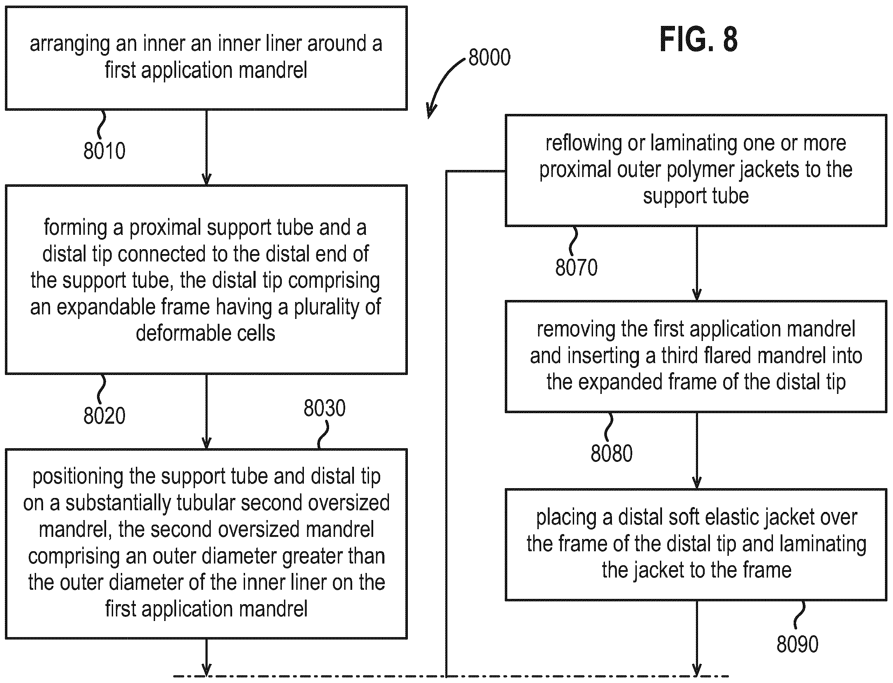

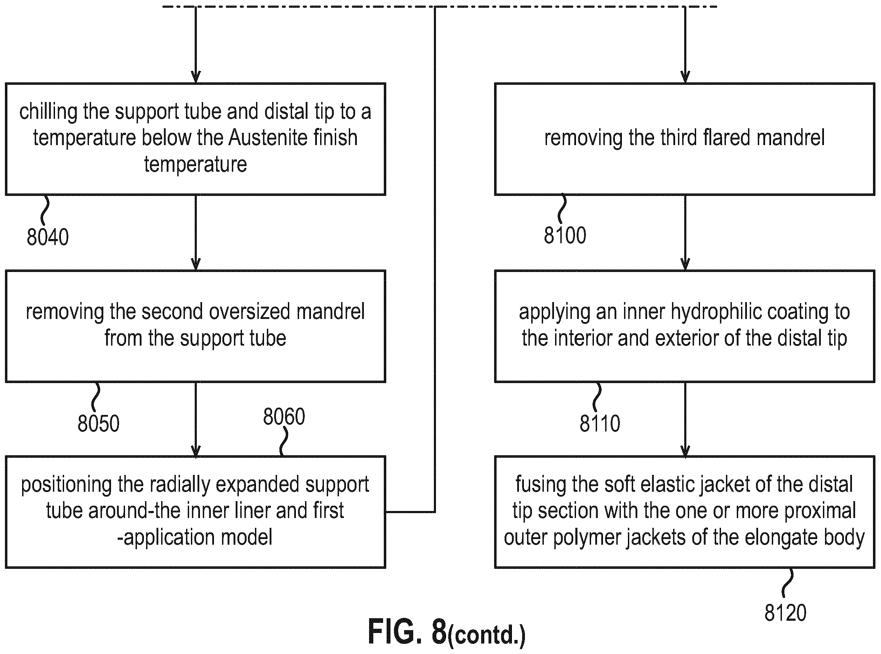

A method for manufacturing a catheter utilizing the disclosed designs is graphically illustrated in FIGs. 7A-7F and further shown in the flow diagram in FIG. 8. FIG. 7A shows a low friction liner 160 positioned on a supporting mandrel 510. The mandrel can often be silver-plated copper (SPC) as is commonly used for these applications. Alternatively, especially ductile materials (such as PEEK) can be used which stretch to neck down in diameter so that the mandrel can be removed after completion of the catheter assembly. Further mandrel materials can include nylon coated copper or nylon coated steel.

The inner liner 160 can be a lubricious, low friction material such as PTFE to aid in a clot being pulled proximally through the catheter with aspiration and/or a clot retrieval device. The liner can also help with the initial delivery of these and other devices to the target site through the lumen of the catheter.

A laser cut elongate body support tube 110 with a distal radially expandable support frame 210 is formed in FIG. 7B. The elongate body can have a plurality of ribs 118 extending along a longitudinal axis 111 between a proximal end 112 and a distal end 114. In some examples, the elongate body 110 and tip support frame 210 can be cut from a hypotube of NiTi or another shape memory superelastic alloy so that the solid state phase transformations can be designed to dictate the constrained and unconstrained diameters of the frame.

In many cases, the ribs 118 can have circumferential discontinuities or seams allowing the frame of the elongate body 110 to expand radially in an elastic fashion. This expansion allows the elongate body to have a nominal inner diameter roughly the same size (or even slightly smaller) than the outer diameter of the liner 160, but the capability to expand over the liner during assembly. Ribs 118 can be arranged and varied along the longitudinal axis 111 such that the elongate body 110 has good pushability and column strength near the proximal end 112 and excellent lateral flexibility near the distal end 114.

The support frame 210 can have deformable cells configured to have a longitudinally elongated, nearly flat shape when the frame is collapsed for delivery through an outer guide catheter and/or sheath. The frame can be heat set so the cells spring to a radially elongated shape when deployed, taking up a much greater proportion of the frame circumference.

In FIG. 7C, the elongate body 110 is radially expanded and slid with the support frame 210 over an oversized mandrel 520. The oversized mandrel 20 can have, for example, a diameter at least 0.13mm (0.005 inches) greater than that of the inner liner 160 on the application mandrel 510. The elongate body 110 and support frame 210 can then be chilled to a lower temperature (ideally close to or below the Martensite Finish (M_{f}) temperature) to transform the material to the martensitic phase. In another example, the structure can be chilled first and then expanded over the oversized mandrel 520. If kept chilled, the reversible solid state transformation to martensite can allow the elongate body 110 to maintain its radially expanded shape when removed from the oversized mandrel 520.

Alternatively, the chilling steps can be eliminated by disposing a thin outer metal sleeve (not shown) around the oversized mandrel 520. The elongate body 110 can be elastically expanded over the sleeve/oversized mandrel assembly and the oversized mandrel 520 removed. The sleeve constrains the elongate body radially so that it maintains the expanded shape and can be slid over the inner liner 160 on the application mandrel 510. When the sleeve support is removed, the elongate body 110 can contract down onto the inner liner 160.

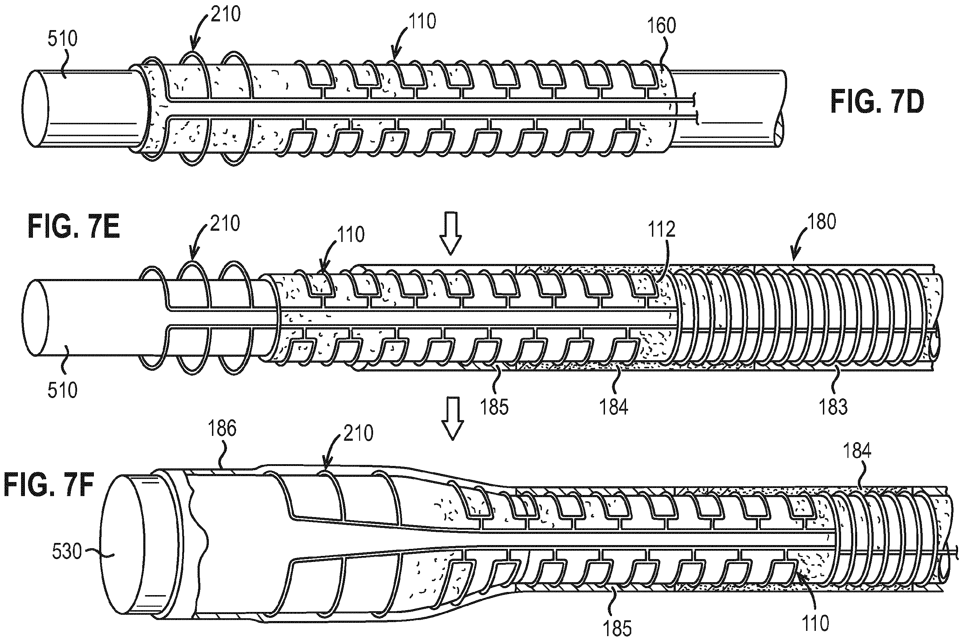

The expanded elongate body 110 and support frame 210 can then be slid over the inner liner 160 on the SPC application mandrel 510 as depicted in FIG. 7D. Without previously expanding the elongate body, this step can generate too much friction to create a reliable and repeatable interface between the body and liner. Once in place and concentric with the liner 160, an outer polymer layer 180 can be applied over the support tube 100 (FIG. 7E). The layer 180 can be an axial series of separate polymer jacket extrusions which can be reflowed or laminated in place as outer jackets 183, 184, 185. The applied heat can allow the outer polymer to fill the interstitial sites between the rib struts 118 of the support tube. Suitable jacket materials can include elastic polyurethanes such as Chronoprene, which can have a shore hardness of 40A or lower, or silicone elastomers.

Alternatively, the jackets can also be applied in a combination of other ways. Depending on their axial location on the elongate body 110, the jackets 183, 184, 185 can be dip coated, sprayed, electro spun, and/or plasma deposited onto the supporting frame. In other examples, the jackets can be a straight extrusion or extruded and post-formed onto the expanding tip and catheter body.

In order to allow for smooth delivery of the clot retrieval catheter 100 through an outer catheter, the outer surface of the polymer layer 180 can be coated with a low-friction or lubricious material, such as PTFE or commercially available lubricious coatings such as offered by Surmodics, Harland, Biocoat or Covalon. Similarly, the inner surface of the catheter shaft 220 can also be coated with the same or similar low-friction material for the passage of auxiliary devices and to aid in a captured clot being drawing proximally through the catheter 100 with aspiration and/or a clot retrieval device.

FIG. 7F shows a flared stepped mandrel 530 back loaded into the support frame 210 after the application mandrel 510 has been removed from the pre-reflowed elongate body 110 with proximal jackets 183, 184, 185. A soft, elastic polymer tip jacket 186 extrusion can be threaded or stretched over the stepped mandrel tool 530 and pushed over the flared section to expand the undersized material of the extrusion. Alternatively, the jacket 186 material can be sized to fit over the stepped portion of the mandrel 530.

In some instances, a temporary sleeve of PTFE or other suitable low friction material can be applied over the shaft of the elongate body 110 and used to control and arrest the proximal flow of the distal tip jacket 186 as it is reflowed into place. In other examples, a layer of heat shrink can be positioned around the jacket 186 extrusion to better conform the material to the contours of the stepped mandrel during reflow or lamination. Once complete, stepped mandrel 530 can be removed from the assembly, and if necessary, any excess material can be trimmed away to ensure the desired catheter profile is attained.

A similar process is outlined in the method flow diagram in FIG. 8. The method steps can be implemented for any of the example devices or suitable alternatives described herein and known to one of ordinary skill in the art. The method can have some or all the steps described, and in many cases, steps can be performed in a different order than that disclosed below.

Referring to FIG. 8, the method 8000 can have the step 8010 of arranging an inner liner around a first application mandrel. The mandrel can be used to give structure during manufacturing and define what can be the inner lumen of the catheter. The mandrel can be silver-plated copper (SPC) or other commonly used materials and sized to have an outer diameter such that the resulting catheter has a bore larger than many contemporary aspiration catheters (at least 1.78mm (0.070 inches)). The liner can be PTFE or a similar low friction material. A strike layer can also be included to better adhere the inner liner to subsequent layers of the catheter shaft.

Step 8020 can then involve forming a laser cut elongate body and expandable support frame structure for the distal tip as described previously herein. The elongate body and support frame can be cut from single continuous hypotube, which can be but is not limited to Nitinol or another shape memory superelastic alloy. The frame of the distal tip can have deformable cells with struts that can collapse to be almost flat (forming an angle with the longitudinal axis that is less than 30 degrees) for easy of deliverability. Similarly, the cells can expand circumferentially to a very broad angle when the support frame is expanded for enhanced hoop strength

In step 8030, the elongate body and support frame of the distal tip can be positioned on a substantially tubular second oversized mandrel. In some examples, the oversized mandrel can be sized so that the expanded inner diameter of the support tube frame is slightly larger than the outer diameter of the inner liner on the application mandrel. For instance, the outer diameter can be approximately 0.07-0.13mm (0.003 - 0.005 inches) larger than the outer diameter of the liner.

For step 8040, once the elongate body is expanded on the oversized mandrel, it can be chilled to a temperature at least below the A_{f} temperature, and ideally close to or below the M_{f} temperature of the material to induce a phase change to martensite. The martensitic phase is thermodynamically stable, so the elongate body can be kept chilled and can retain its expanded state when the second oversized mandrel is removed in step 8050.

The expanded elongate body and distal support frame can then be slid over and positioned around the inner liner on the first application mandrel in step 8060. A series of outer polymer jackets of varying durometer hardness can then be reflowed to the support tube (step 8070). The inner liner and outer jackets can cease at the proximal end of the support frame, or some distance proximal of the proximal end. The jackets can be in an axial series, a radial series, or some combination. The flow of the jacket materials can allow them to encapsulate the ribs struts and/or braids of the elongate body and bond with the inner liner. The first application mandrel can be removed in step 8080 and a third flared or stepped mandrel can be fed proximally into the expanded support frame of the distal tip.

Step 8090 can involve threading or stretching a soft distal polymeric jacket over the support frame. The distal polymeric jacket can extend proximally to the distal edge of the proximal outer jackets of the elongate body and overhang at least several millimeters distally beyond the distal end of the support frame. The supporting frame can allow very soft materials to be used for their atraumatic properties, such as those with a hardness below 30 Shore D (approximately 80 Shore A). A heat shrink sleeve consisting of FEP or another suitable copolymer can enable the distal jacket extrusion to conform to the contours of the flared mandrel when the heat from the reflow process is applied. The step can then involve reflowing or laminating the distal polymeric jacket to the support frame (and a distal portion of the catheter shaft if desired). Once the jacket is applied to the support frame, the third flared mandrel can be removed in step 8100.

If desired, an inner hydrophilic coating can be applied to the distal tip in step 8110. A process such as dip coating can be used to apply the coating to both the inner and outer surfaces after removal of the flared mandrel. A further post-processing reflow step (step 8120) can also be used to apply additional material or flow existing material to fuse the distal outer jacket of the support frame to the proximal jackets of the elongate body.

Depending on the design, some elongate bodies may not be expandable from their nominal diameter to fit over the low friction polymeric inner liner and strike layer on the application mandrel. These can include a slotted tube, or a puzzle cut tube as seen in FIGs. 5A and 5B. In these instances, the method can involve laser cutting a proximal elongate body support tube and distal expandable tip from a hypotube. The low friction liner sleeve can then be folded and positioned within at least a portion of the proximal laser cut elongate body. The liner can be a material like PTFE and the strike layer can be manufactured from a second, higher friction material. A heated mandrel with an enlarged plug end can then be drawn through the inner liner to expand and adhere the liner to the inner surface of the elongate body. Similar procedural steps to those above, including the use of a flared mandrel, can be used to apply the outer polymer jackets to the elongate body and support frame.

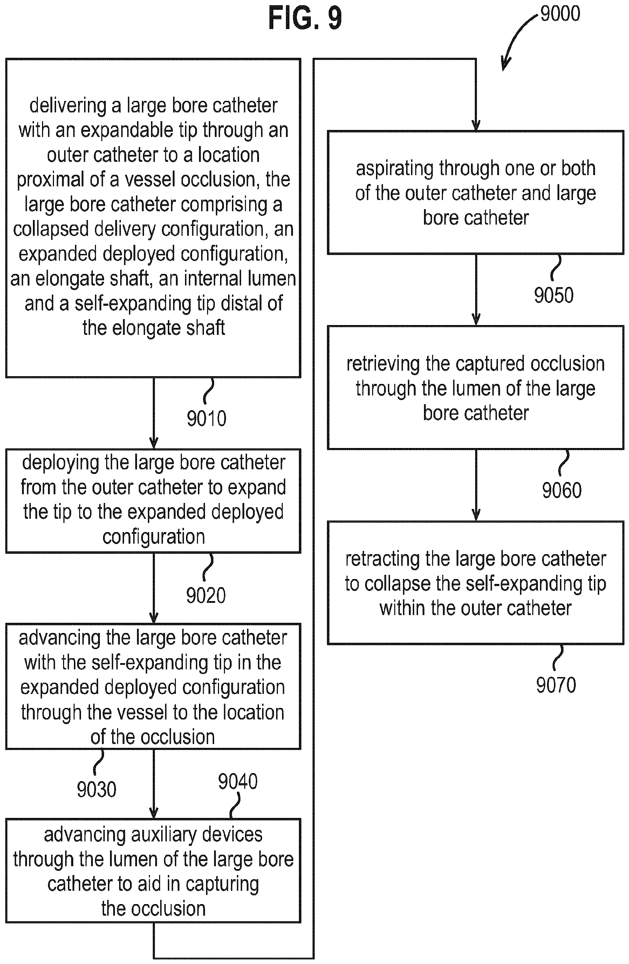

A method not part of the invention for using a catheter of the present disclosure to clear an occlusion from a body vessel is shown in FIG. 9. In step 9010, a large bore catheter with an expandable tip can be delivered through an outer catheter to a location proximal of a vessel occlusion. The outer catheter is typically placed as close to the occlusive clot as practical, but the location can depend on the destination vessel size and the relative tortuosity of the vasculature needed to reach it. For example, in the case of a middle cerebral artery occlusion, the outer catheter might be placed in the internal carotid artery proximal of the carotid siphon. If for example the target occlusion is in an M1 vessel, a typical guide or outer sheath will need to be maintained in a position well proximal of these vessel diameters.

The large bore catheter can have a collapsed delivery configuration, an expanded deployed configuration, an elongate shaft, an internal lumen, and an expandable tip at the distal end of the elongate shaft. A support frame of the expandable tip can have deformable cells with struts that can collapse to be as flat as possible (nearly parallel with the longitudinal axis) for easy of deliverability. The deformable cells can also elongate circumferentially in the expanded deployed configuration during the process of dislodging and capturing a clot to increase the radial size of the expandable tip for more efficient aspiration and improved interaction with the clot upon ingestion.

Step 9020 can include deploying the large bore catheter beyond the distal end of the outer catheter to expand the tip to the expanded deployed configuration. The frame of the tip can be of a superelastic alloy and heat set to the expanded shape such that the deformable cells are effectively spring loaded when constrained within the outer catheter during delivery. Since the size of the expanded tip is smaller than the target vessel diameter, the large bore catheter can be advanced distally through the vessels to the location of the occlusion in the expanded configuration in step 9030. This gives the catheter the advantage of being able to deploy a distance proximal (for example, 3-5 cm) of the occlusion location for improved distal access, as opposed to directly at the occlusion like many other expandable devices. The tip can have an atraumatic vessel crossing profile and a soft outer jacket to prevent damage and/or snagging on bifurcations. The circumferentially elongated deformable cells can minimize further expansion of the tip as it is advanced through vessels in the expanded state.

Once the target is reached, the occlusion can be aspirated and drawn into the tip and lumen of the large bore catheter. If necessary, auxiliary devices such as microcatheters and clot retrieval devices can be advanced through the lumen of the large bore catheter and used against obstinate clots (step 9040). Aspiration can be directed through one or both of the outer catheter and large bore catheter (step 9050). The expanded tip of the large bore catheter is able to direct more of the suction distally to the clot, as opposed to fluid proximal of the tip.

Once the occlusion has been dislodged from the vessel walls, step 9060 can involve progressively compressing the clot through the funnel-shape of the expandable tip of the large bore catheter and through the lumen into a cannister or syringe. The large bore catheter can remain in position during this step to maintain access to the target site. Contrast can then be injected to determine the extent to which the vessel is patent. Clot retrieval devices may be rinsed in saline and gently cleaned before being reloaded into the microcatheter for additional passes, if required. When the vessel has been satisfactorily cleared, the large bore catheter can be retracted in step 9070 to collapse the expandable tip within the outer catheter. The deformable cells of the tip can transition back to the folded, axially-elongated state to facilitate consistent wrap down of the tip.

The invention is not necessarily limited to the examples described, which can be varied in construction and detail. The terms "distal" and "proximal" are used throughout the preceding description and are meant to refer to a positions and directions relative to a treating physician. As such, "distal" or distally" refer to a position distant to or a direction away from the physician. Similarly, "proximal" or "proximally" refer to a position near or a direction towards the physician. Furthermore, the singular forms "a," "an," and "the" include plural referents unless the context clearly dictates otherwise.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

## Claims

1. A large bore catheter (100) comprising:
a longitudinal axis;
an elongate body (110) comprising a lumen, a proximal end, and a distal end;
a support frame (210) at the distal end of the elongate body having a collapsed delivery configuration and an expanded deployed configuration, the support frame (210) comprising a framework of struts comprising one or more hoop segments (213) and a plurality of deformable cells (220), the deformable cells (220) having apices, creases, or other biasing features such that the deformable cells (220) are configured to assume an axially extended profile when the support frame (210) is in the collapsed delivery configuration and expand to a radially extended profile when the support frame (210) is in the expanded deployed configuration;
the expanded deployed configuration of the support frame (210) sized to have a larger inner diameter than the inner diameter of an outer sheath,
**characterised by**
the one or more hoop segments (213) and the plurality of deformable cells (220) accounting for a percentage of the circumference of the support frame (210), and wherein the one or more hoop segments (213) are of fixed size such that the percentage accounted for by the one or more hoop segments (213) is greater in the collapsed delivery configuration than the percentage in the expanded deployed configuration, the deformable cells (220) taking a percentage in the expanded deployed configuration that is larger than in the collapsed delivery configuration.

2. The catheter of claim 1, the struts of the deformable cells (220) comprising an angle with the longitudinal axis of i) less than 30 degrees in the collapsed delivery configuration, or ii) greater than 45 degrees in the expanded deployed configuration.

3. The catheter of claim 1 or claim 2, the support frame (210) further comprising i) one axial row of deformable cells (220), or ii) two axial rows of deformable cells (220).

4. The catheter of any one of the preceding claims, the deformable cells (220) comprising less than 30% of the circumference of the support frame (210) when the frame is in the collapsed delivery configuration.

5. The catheter of any one of the preceding claims, the support frame (210) further comprising a longitudinal length sized to be less than three times an inner diameter of the elongate body.

6. The catheter of any one of the preceding claims, the support frame (210) further comprising an inner diameter of approximately 1.78mm (0.070 inches) in the collapsed delivery configuration and a maximum inner diameter in a range of approximately 2.03 - 3.05 mm (0.080 - 0.120 inches) in the expanded deployed configuration.

7. The catheter of any one of the preceding claims, the difference between the inner diameter of the support frame (210) in the expanded deployed configuration and the inner diameter of the support frame (210) in the collapsed delivery configuration being more than 10% of the inner diameter in the collapsed delivery configuration.

8. The catheter of any one of the preceding claims, the struts of the deformable cells (220) comprising a first arc length in the collapsed delivery configuration less than a second arc length in the expanded deployed configuration.

9. The catheter of any one of the preceding claims, the support frame (210) further comprising one or more connecting spines (218) connecting the hoop segments (213) of the support frame (210) with the elongate body (110).

10. The catheter of any one of the preceding claims, the support frame (210) comprising a maximum outer diameter in the expanded deployed configuration less than an inner diameter of a target vessel at a treatment site.

11. The catheter of any one of the preceding claims, the struts of the deformable cells (220) forming a cell angle with the longitudinal axis of greater than 70 degrees in the expanded deployed configuration.

12. The catheter of any one of the preceding claims, the struts of at least a portion of the support frame (210) forming a flare angle with the longitudinal axis, the flare angle configured to be approximately zero degrees in the collapsed delivery configuration and approximately 90 degrees in the expanded deployed configuration.

13. A method for manufacturing a catheter of any one of the preceding claims, the method comprising the steps of:
arranging an inner liner (160) around a first application mandrel (510);
forming an elongate body (110) disposed around a longitudinal axis and a support frame (210) connected to the distal end of the elongate body (110), the support frame (210)
comprising a framework of struts comprising one or more hoop segments (213) and a plurality of deformable cells (220) configured to assume an axially extended profile forming an acute cell angle with the longitudinal axis when the support frame (210) is in the collapsed delivery configuration and expand to a radially extended profile when the support frame (210) is in the expanded deployed configuration;
the expanded deployed configuration of the support frame (210) sized to have a larger inner diameter than the inner diameter of an outer sheath, the one or more hoop segments (213) accounting for a percentage of the circumference of the support frame (210), and wherein the one or more hoop segments (213) are of fixed size such that the percentage is greater in the collapsed delivery configuration than the percentage in the expanded deployed configuration;
positioning the elongate body (110) and support body (210) on a substantially tubular second oversized mandrel (520), the second oversized mandrel (520) comprising an outer diameter greater than the outer diameter of the inner liner on the first application mandrel (510);
chilling the elongate body (110) and support frame (210) to a temperature below the Austenite finish temperature;
removing the second oversized mandrel (520) from the elongate body (110);
positioning the elongate body (110) around the inner liner (160) and first application mandrel (510);
reflowing or laminating one or more proximal outer polymer jackets to the elongate body (110);
removing the first application mandrel (510) and inserting a third flared mandrel (530) into the expanded frame of the support body (210);
placing a distal soft elastic jacket over the frame of the support body (210) and laminating the jacket to the elongate body (110) and support body (210); and
removing the third flared mandrel (530).

14. The method of claim 13, further comprising the step of applying an inner hydrophilic coating to the interior and exterior of the support body (210), optionally further comprising the step of fusing the soft elastic jacket of the support body (210) with the one or more proximal outer polymer jackets of the elongate body (110).

## Patentansprüche

1. Katheter (100) mit großer Bohrung, umfassend:
eine Längsachse;
einen länglichen Körper (110), umfassend ein Lumen, ein proximales Ende und ein distales Ende;
einen Stützrahmen (210) an dem distalen Ende des länglichen Körpers, der eine zusammengeklappte Zuführkonfiguration und eine expandierte entfaltete Konfiguration aufweist, der Stützrahmen (210) umfassend ein Strebengerüst, umfassend ein oder mehrere Reifensegmente (213) und eine Vielzahl von verformbaren Zellen (220), wobei die verformbaren Zellen (220) Spitzen, Falten oder andere Vorspannmerkmale derart aufweisen, dass die verformbaren Zellen (220) konfiguriert sind, um ein axial ausgedehntes Profil anzunehmen, wenn sich der Stützrahmen (210) in der zusammengeklappten Zuführkonfiguration befindet, und sich zu einem radial ausgedehnten Profil zu expandieren, wenn sich der Stützrahmen (210) in der expandierten entfalteten Konfiguration befindet;
wobei die expandierte entfaltete Konfiguration des Stützrahmens (210) dimensioniert ist, um einen größeren Innendurchmesser als der Innendurchmesser einer Außenhülle aufzuweisen,
**dadurch gekennzeichnet, dass** das eine oder die mehreren Reifensegmente (213) und die Vielzahl von verformbaren Zellen (220) einen Prozentsatz des Umfangs des Stützrahmens (210) ausmachen, und wobei das eine oder die mehreren Reifensegmente (213) von fester Dimension derart sind, dass der Prozentsatz, den das eine oder die mehreren Reifensegmente (213) ausmachen, in der zusammengeklappten Zuführkonfiguration höher als der Prozentsatz in der expandierten entfalteten Konfiguration ist, wobei die verformbaren Zellen (220) in der expandierten entfalteten Konfiguration einen Prozentsatz einnehmen, der größer als in der zusammengeklappten Zuführkonfiguration ist.

2. Katheter nach Anspruch 1, die Streben der verformbaren Zellen (220) umfassend einen Winkel mit der Längsachse von i) kleiner als 30 Grad in der zusammengeklappten Zuführkonfiguration oder ii) höher als 45 Grad in der expandierten entfalteten Konfiguration.

3. Katheter nach Anspruch 1 oder 2, der Stützrahmen (210) ferner umfassend i) eine axiale Reihe von verformbaren Zellen (220) oder ii) zwei axiale Reihen von verformbaren Zellen (220).

4. Katheter nach einem der vorstehenden Ansprüche, die verformbaren Zellen (220) umfassend weniger als 30 % des Umfangs des Stützrahmens (210), wenn sich der Rahmen in der zusammengeklappten Zuführkonfiguration befindet.

5. Katheter nach einem der vorstehenden Ansprüche, der Stützrahmen (210) ferner umfassend eine Längslänge, die dimensioniert ist, um kleiner als ein Dreifaches eines Innendurchmessers des länglichen Körpers zu sein.

6. Katheter nach einem der vorstehenden Ansprüche, der Stützrahmen (210) ferner umfassend einen Innendurchmesser von etwa 1,78 mm (0,070 Zoll) in der zusammengeklappten Zuführkonfiguration und einen maximalen Innendurchmesser in einem Bereich von etwa 2,03-3,05 mm (0,080 -0,120 Zoll) in der expandierten entfalteten Konfiguration.

7. Katheter nach einem der vorstehenden Ansprüche, wobei der Unterschied zwischen dem Innendurchmesser des Stützrahmens (210) in der expandierten entfalteten Konfiguration und dem Innendurchmesser des Stützrahmens (210) in der zusammengeklappten Zuführkonfiguration mehr als 10 % des Innendurchmessers in der zusammengeklappten Zuführkonfiguration beträgt.

8. Katheter nach einem der vorstehenden Ansprüche, die Streben der verformbaren Zellen (220) umfassend eine erste Bogenlänge in der zusammengeklappten Zuführkonfiguration, die kleiner als eine zweite Bogenlänge in der expandierten entfalteten Konfiguration ist.

9. Katheter nach einem der vorstehenden Ansprüche, der Stützrahmen (210) ferner umfassend einen oder mehrere Verbindungsrücken (218), die die Reifensegmente (213) des Stützrahmens (210) mit dem länglichen Körper (110) verbinden.

10. Katheter nach einem der vorstehenden Ansprüche, der Stützrahmen (210) umfassend einen maximalen Außendurchmesser in der expandierten entfalteten Konfiguration, der kleiner als ein Innendurchmesser eines Zielgefäßes an einer Behandlungsstelle ist.

11. Katheter nach einem der vorstehenden Ansprüche, wobei die Streben der verformbaren Zellen (220) einen Zellwinkel mit der Längsachse von höher als 70 Grad in der expandierten entfalteten Konfiguration formen.

12. Katheter nach einem der vorstehenden Ansprüche, wobei die Streben von mindestens einem Abschnitt des Stützrahmens (210) einen Aufweitungswinkel mit der Längsachse formen, wobei der Aufweitungswinkel konfiguriert ist, um etwa null Grad in der zusammengeklappten Zuführkonfiguration und etwa 90 Grad in der expandierten entfalteten Konfiguration zu betragen.

13. Verfahren zum Herstellen eines Katheters nach einem der vorstehenden Ansprüche, das Verfahren umfassend die Schritte:
Anordnen einer Innenauskleidung (160) um einen ersten Applikationsdorn (510) herum;
Formen eines länglichen Körpers (110), der um eine Längsachse herum eingerichtet ist, und eines Stützrahmens (210), der mit dem distalen Ende des länglichen Körpers (110) verbunden ist, der Stützrahmen (210)
umfassend ein Strebengerüst, umfassend ein oder mehrere Reifensegmente (213) und eine Vielzahl von verformbaren Zellen (220), die konfiguriert sind, um ein axial ausgedehntes Profil anzunehmen, das einen spitzen Zellwinkel mit der Längsachse formt, wenn sich der Stützrahmen (210) in der zusammengeklappten Zuführkonfiguration befindet, und sich zu einem radial ausgedehnten Profil expandiert, wenn sich der Stützrahmen (210) in der expandierten entfalteten Konfiguration befindet;
wobei die expandierte entfaltete Konfiguration des Stützrahmens (210) dimensioniert ist, um einen größeren Innendurchmesser als der Innendurchmesser einer Außenhülle aufzuweisen, wobei das eine oder die mehreren Reifensegmente (213) einen Prozentsatz des Umfangs des Stützrahmens (210) ausmachen und wobei das eine oder die mehreren Reifensegmente (213) von fester Dimension derart sind, dass der Prozentsatz in der zusammengeklappten Zuführkonfiguration höher als der Prozentsatz in der expandierten entfalteten Konfiguration ist;
Positionieren des länglichen Körpers (110) und eines Stützkörpers (210) an einem im Wesentlichen röhrenförmigen zweiten überdimensionierten Dorn (520), der zweite überdimensionierte Dorn (520) umfassend einen Außendurchmesser, der höher als der Außendurchmesser der Innenauskleidung an dem ersten Applikationsdorn (510) ist;
Abkühlen des länglichen Körpers (110) und des Stützrahmens (210) auf eine Temperatur unterhalb der Austenitendtemperatur;
Entfernen des zweiten überdimensionierten Dorns (520) aus dem länglichen Körper (110);
Positionieren des länglichen Körpers (110) um die Innenauskleidung (160) und den ersten Applikationsdorn (510) herum;
Aufschmelzen oder Laminieren eines oder mehrerer proximaler Außenpolymermäntel auf den länglichen Körper (110);
Entfernen des ersten Applikationsdorns (510) und Einführen eines dritten aufgeweiteten Dorns (530) in den expandierten Rahmen des Stützkörpers (210);
Platzieren eines distalen weichen elastischen Mantels über dem Rahmen des Stützkörpers (210) und Laminieren des Mantels auf den länglichen Körper (110) und den Stützkörper (210); und
Entfernen des dritten aufgeweiteten Dorns (530).

14. Verfahren nach Anspruch 13, ferner umfassend den Schritt eines Applizierens einer hydrophilen Innenbeschichtung auf das Innere und Äußere des Stützkörpers (210), optional ferner umfassend den Schritt eines Verschmelzens des weichen elastischen Mantels des Stützkörpers (210) mit dem einen oder den mehreren proximalen Außenpolymermänteln des länglichen Körpers (110).

## Revendications

1. Cathéter à gros calibre (100) comprenant :
un axe longitudinal ;
un corps allongé (110) comprenant une lumière, une extrémité proximale, et une extrémité distale ;
un cadre de support (210) au niveau de l'extrémité distale du corps allongé ayant une configuration d'administration repliée et une configuration déployée élargie, le cadre de support (210) comprenant un cadre d'entretoises comprenant un ou plusieurs segments de cerceau (213) et une pluralité de cellules déformables (220), les cellules déformables (220) ayant des sommets, des plis, ou d'autres caractéristiques de sollicitation, de telle sorte que les cellules déformables (220) sont conçues pour adopter un profil axialement étendu lorsque le cadre de support (210) est dans la configuration d'administration repliée et pour s'élargir à un profil radialement étendu lorsque le cadre de support (210) est dans la configuration déployée élargie ;
la configuration déployée élargie du cadre de support (210) étant dimensionnée pour avoir un diamètre interne plus grand que le diamètre interne d'une enveloppe externe, **caractérisé en ce que** le ou les segments de cerceau (213) et la pluralité de cellules déformables (220) représentent un pourcentage de la circonférence du cadre de support (210), et dans lequel le ou les segments de cerceau (213) sont de taille fixe de telle sorte que le pourcentage représenté par le ou les segments de cerceau (213) est plus grand dans la configuration d'administration repliée que le pourcentage dans la configuration déployée élargie, les cellules déformables (220) prenant un pourcentage dans la configuration déployée élargie qui est plus grand que dans la configuration d'administration repliée.

2. Cathéter selon la revendication 1, les entretoises des cellules déformables (220) comprenant un angle avec l'axe longitudinal de i) moins de 30 degrés dans la configuration d'administration repliée, ou ii) plus de 45 degrés dans la configuration déployée élargie.

3. Cathéter selon la revendication 1 ou la revendication 2, le cadre de support (210) comprenant en outre i) une rangée axiale de cellules déformables (220), ou ii) deux rangées axiales de cellules déformables (220).

4. Cathéter selon l'une quelconque des revendications précédentes, les cellules déformables (220) représentant moins de 30 % de la circonférence du cadre de support (210) lorsque le cadre est dans la configuration d'administration repliée.

5. Cathéter selon l'une quelconque des revendications précédentes, le cadre de support (210) comprenant en outre une longueur longitudinale dimensionnée pour être inférieure à trois fois un diamètre interne du corps allongé.

6. Cathéter selon l'une quelconque des revendications précédentes, le cadre de support (210) comprenant en outre un diamètre interne d'approximativement 1,78 mm (0,070 pouce) dans la configuration d'administration repliée et un diamètre interne maximal dans une plage d'approximativement 2,03 - 3,05 mm (0,080 - 0,120 pouce) dans la configuration déployée élargie.

7. Cathéter selon l'une quelconque des revendications précédentes, la différence entre le diamètre interne du cadre de support (210) dans la configuration déployée élargie et le diamètre interne du cadre de support (210) dans la configuration d'administration repliée étant supérieure à 10 % du diamètre interne dans la configuration d'administration repliée.

8. Cathéter selon l'une quelconque des revendications précédentes, les entretoises des cellules déformables (220) comprenant une première longueur d'arc dans la configuration d'administration repliée inférieure à une seconde longueur d'arc dans la configuration déployée élargie.

9. Cathéter selon l'une quelconque des revendications précédentes, le cadre de support (210) comprenant en outre une ou plusieurs tiges de liaison (218) reliant les segments de cerceau (213) du cadre de support (210) au corps allongé (110).

10. Cathéter selon l'une quelconque des revendications précédentes, le cadre de support (210) comprenant un diamètre externe maximal dans la configuration déployée élargie inférieur à un diamètre interne d'un vaisseau cible au niveau d'un site de traitement.

11. Cathéter selon l'une quelconque des revendications précédentes, les entretoises des cellules déformables (220) formant un angle de cellule avec l'axe longitudinal supérieur à 70 degrés dans la configuration déployée élargie.

12. Cathéter selon l'une quelconque des revendications précédentes, les entretoises d'au moins une partie du cadre de support (210) formant un angle d'évasement avec l'axe longitudinal, l'angle d'évasement étant configuré pour être approximativement de zéro degré dans la configuration d'administration repliée et approximativement de 90 degrés dans la configuration déployée élargie.

13. Procédé de fabrication d'un cathéter selon l'une quelconque des revendications précédentes, le procédé comprenant les étapes consistant à :
disposer un revêtement interne (160) autour d'un premier mandrin d'application (510) ;
former un corps allongé (110) disposé autour d'un axe longitudinal et un cadre de support (210) relié à l'extrémité distale du corps allongé (110), le cadre de support (210)
comprenant un cadre d'entretoises comprenant un ou plusieurs segments de cerceau (213) et une pluralité de cellules déformables (220) conçues pour adopter un profil axialement étendu formant un angle de cellule aigu avec l'axe longitudinal lorsque le cadre de support (210) est dans la configuration d'administration repliée et pour s'élargir à un profil radialement étendu lorsque le cadre de support (210) est dans la configuration déployée élargie ;
la configuration déployée élargie du cadre de support (210) étant dimensionnée pour avoir un diamètre interne plus grand que le diamètre interne d'une enveloppe externe, le ou les segments de cerceau (213) représentant un pourcentage de la circonférence du cadre de support (210), et dans lequel le ou les segments de cerceau (213) sont de taille fixe de telle sorte que le pourcentage est plus grand dans la configuration d'administration repliée que le pourcentage dans la configuration déployée élargie ;
positionner le corps allongé (110) et le corps de support (210) sur un deuxième mandrin surdimensionné (520) sensiblement tubulaire, le deuxième mandrin surdimensionné (520) comprenant un diamètre externe supérieur au diamètre externe du revêtement interne sur le premier mandrin d'application (510) ;
refroidir le corps allongé (110) et le cadre de support (210) à une température inférieure à la température de finition de l'austénite ;
retirer le deuxième mandrin surdimensionné (520) du corps allongé (110) ;
positionner le corps allongé (110) autour du revêtement interne (160) et du premier mandrin d'application (510) ;
refusionner ou laminer une ou plusieurs enveloppes externes proximales en polymère sur le corps allongé (110) ;
retirer le premier mandrin d'application (510) et insérer un troisième mandrin évasé (530) dans le cadre élargi du corps de support (210) ;
placer une enveloppe élastique souple distale sur le cadre du corps de support (210) et laminer la enveloppe sur le corps allongé (110) et le corps de support (210) ; et
retirer le troisième mandrin évasé (530).

14. Procédé selon la revendication 13, comprenant en outre l'étape consistant à appliquer un revêtement hydrophile interne à l'intérieur et à l'extérieur du corps de support (210), comprenant facultativement en outre l'étape consistant à fusionner l'enveloppe élastique souple du corps de support (210) avec la ou les enveloppes externes proximales en polymère du corps allongé (110).
